Europäisches Patentamt

European Patent Office

Office européen des brevets

(19)

(11) Publication number: **0 264 067**

**A2**

(12) # EUROPEAN PATENT APPLICATION

(21) Application number: **87114701.3**

(22) Date of filing: **08.10.87**

(51) Int. Cl.⁴: **C12N 15/00** , C12P 21/02 ,
//C12N9/04

The applicant has filed a statement in accordance with Rule 28 (4) EPC (issue of a sample only to an expert). Accession number(s) of the deposit(s): ATCC 67225 - 67226 - 67224.

(30) Priority: **08.10.86 US 917101**

(43) Date of publication of application:
**20.04.88 Bulletin 88/16**

(84) Designated Contracting States:
**AT BE CH DE ES FR GB GR IT LI LU NL SE**

(71) Applicant: **THE GENERAL HOSPITAL CORPORATION**
**55 Fruit Street**
**Boston MA 02114(US)**

(72) Inventor: **Shi, Ming-Che**
**23 Cufflin Street**
**Brighton, Mass, 02135(US)**
Inventor: **Goodman, Howard M, Prof.**
**10 The Ledges Road,**
**Newton center, Mass. 02159(US)**

(74) Representative: **Meyer-Dulheuer,**
**Karl-Hermann, Dr. et al**
**HOECHST Aktiengesellschaft Zentrale**
**Patentabteilung Postfach 80 03 20**
**D-6230 Frankfurt/Main 80(DE)**

(54) Production of glyceraldehyde-3 phosphate dehydrogenase.

(57) Plant glyceraldehyde phosphate dehydrogenase produced from recombinant DNA has been disclosed. A host transformed with a recombinant DNA molecule which contains a plant derived sequence for glyceraldehyde phosphate dehydrogenase has also been disclosed.

EP 0 264 067 A2

# PRODUCTION OF GLYCERALDEHYDE-3 PHOSPHATE DEHYDROGENASE

## BACKGROUND OF THE INVENTION

### Field of the Invention

The present invention is directed to the production of glyceraldehyde-3-phosphate dehydrogenase by recombinant DNA technology.

### Brief Description of the Background Art

Glyceraldehyde-3-phosphate dehydrogenase is an enzyme which functions in the glycolytic pathway, gluconeogenesis ano carbon fixation. It is found in the cytosol of plants, animals and bacteria where it catalyzes the conversion of glyceraldehyde-3-phosphate to 1,3 diphosphoglycerate, and in chloroplasts where it catalyzes this reaction in reverse as part of the Calvin cycle.

Three bacterial GAPDH sequences have been determined: one from the mesophilic bacterium, E. coli - (Branlant and Branlant, Eur. J. Biochem., 150:61 1985), and two from thermophilic bacteria Bacillus stearothermophilus (Walker et al., Eur. J. Biochem., 108:549, 1980a) and Thermus aquaticus(Walker et al ., Eur. J. Biochem., 108:581, 1980b). The E. coli enzyme was found to be more homologous to eukaryotic cytoso lic GAPDHs than to those of thermophilic bacteria (Branlant and Branlant, 1985). Based on the sequence comparison and hydrophobicity of the S loop (residues 178-201; see Walker et al., 1980a/b; Branlant and Branlant, 1985), it was concluded that GAPDH evolved along two independent lines according to its thermostability since it has been suggested that the hydrophobicity of the S loops, which form the core of the tetramer, determine the thermostability of the holoenzyme (Walker et al ., 1980b). Therefore, the amino acid sequence differences observed between eukaryotic and thermophilic bacterial GAPDHs reflect a difference between mesophilic and thermophilic organisms.

Higher plants possess two forms of each glycolytic enzyme; the cytosolic enzymes function in glycolysis, while the chloroplast enzymes are involved in photo-synthetic carbon fixation (Gottlieb, Science, 216:373, 1982). Both cytosolic and chloroplast glycolytic enzymes are encoded by the nuclear genome (Weeden and Gottlieb, J. Hered., 71:392, 1980; Gottlieb, 1982; Cerff and Kloppstech, Proc. Natl Acad. Sci. (USA), 79:7624, 1982).

In higher plants, there are two chloroplast GAPDH isozymes (for review see Cerff, Methods in Chloroplast Molecular Biology, eds. Edelman et al., Elsevier/North Holland, Amsterdam, 683, 1982), isozymes I and II, with subunit structures of $A_2B_2$ and $A_4$, respectively, and there is a single cytosolic GAPDH, with the sub-unit structure of $C_4$. Cytosolic GAPDHs are highly conserved between species and across great evolutionary distances (Tso et al., Nuc. Acid Res., 7:2485, 1985a).

The chloroplast is a semiautonomous organelle of plants and the majority of its functions require gene products codes by both chloroplast and nuclear genes. It is of interest to obtain and provide genetic sequences coding for plant GAPDHs, especially chloroplast derived GAPDHS.

## SUMMARY OF THE INVENTION

It is the purpose of this invention to provide a new source of genetically engineered substantially pure glyceraldehyde-3-phosphate dehydrogenases from plant chloroplasts and cytosols which will be available in unlimited supply.

In accordance with the present invention cDNAs for the nuclear genes encoding the chloroplasts (GapA and GapB) and cytosolic (GapC) GAPDHs from Nicotiana tabacum were cloned and characterized.

The invention thus provides genetic sequences coding for these enzymes, vehicles containing the sequences, hosts transformed therewith, and recombinant DNA technological methods of producing the enzyme(s).

## DESCRIPTION OF THE TABLES

Table 1 shows the N-terminal amino acid sequences of tobacco chloroplast GAPDHs, $A_4$ and $A_2B_2$, as determined by automated Edman degradation.

Table 2 shows oligonucleotide probes for GAPDHs based on the amino acid seuqences (see text). The top panel shows the relative positions in the protein and corresponding amino acid sequences which were used to synthesize the oligonucleotide probes. The bottom panel shows the sequences for the four oligonucleotide probes. The positions within the oligonucleotide with four-fold degeneracy are represented by "N."

Table 3 shows the hybridization patterns of cDNA clones from tobacco leaves to the four synthetic oligonucleotides.

Table 4 shows the nucleotide sequences of cDNA clones and deduced amino acid sequences of tobacco GAPDHs. The nucleotide sequences (a) of the coding strand of cDNA clones pG49 (GapA), pG60 (GapB), and pNDAI (GapC) as determined by the dideoxynucleotide chain termination method and the respective deduced amino acid sequences (b) of tobacco GAPDHs are shown. In part an ellipsis (...) has been added to the sequences where appropriate to maximize homologies, coding sequences are in upper case and the 3' untranslated regions in lower case, and the transit (trn) peptide sequences indicated on separate lines to facilitate comparison to part b. In part b, the deduced amino acid sequence in the one letter code of the A polypeptide is shown. Gaps (x) have been added to maximize homologies to the B and C polypeptides; only differences are shown i.e. a blank in either the GapB or GapC line indicates identity to the GapA line. The number of the left indicates the amino acid residue number of the corresponding peptide in the absence of added homology gaps. The ˙ indicates the end of the polypeptide.

## DEFINITIONS

In the description that follows, a number of terms used in recombinant DNA technology are extensively utilized. In order to provide a clear and consistent understanding of the specification and claims, including the scope to be given such terms, the following definitions are provided.

Gene . A DNA sequence which encodes through its template or messenger RNA a sequence of amino acids characteristic of a specific peptide. The term cDNA includes genes from which the intervening sequences have been removed. By the term rDNA is meant a molecule that has been recombined by splicing cDNA or genomic DNA sequences in vitro.

Cloning Vehicle. A plasmid or phage DNA or other DNA sequence which is able to replicate in a host cell which is characterized by one or a small number of endonuclease recognition sites at which such DNA sequences may be cut in a determinable fashion without loss of an essential biological function of the DNA, and which contains a marker suitable for use in the identification of transformed cells. Markers, for example, are tetracycline resistance or ampicillin resistance. The word "vector" is sometimes used for cloning vehicle.

Expression Vehicle. A vehicle similar to a cloning vehicle but which is capable of expressing a given structural gene in a host, normally under control of certain control sequences.

Expression Control Sequence. A sequence of nucleotides that controls or regulates expression of structrual genes when operably linked to those genes. They include the lac system the trp system, major operator and promoter regions of phage lambda, the control region of fd coat protein and other sequences known to control the expression of genes in prokaryotic or eukaryotic cells.

Operator. A DNA sequence capable of interacting with the specific repressor, thereby controlling the functioning of adjacent gene(s).

Promoter. A DNA sequence in which RNA polymerase binds and initiates transcription of an adjacent gene(s).

Host . The term "host" as used in the present invention is meant to include not only prokaryotes, but also, such eukaryotes as yeasts, filamentous fungi, as well as plant and animal cells.

Prokaryote. The term "prokaryote" is meant to include all bacteria which can be transformed with the GAPDH gene for the expression of the GAPDH protein.

Glyceraldehyde-3 Phosphate Dehydrogenase (GAPDH). This term as used throughout the specification and claims is meant to include glyceraldehyde-3 phosphate dehydrogenase from any plant species which has the enzyme activity in an in vivo or in vitro system. The term is also used in this invention to include any analoque, homologue, mutant, isomer or derivative of a naturally occurring GAPDH. The term is also

3

meant to include fragments having less than the naturally-occurring number of amino acids, such as partial fragments of natural GAPDH which retain the activity of plant cytosolic or chloroplast GAPDH. The term is also used to include any product which comprises the sequence of a naturally occurring plant GAPDH or an analogue thereof together with one or more flanking amino acids, which show GAPDH activity.

The term "substantially pure form" when applied to the protein of the invention means that the protein is essentially free of other cellular proteins with which the enzyme produced by the invention is normally associated in nature.

## BRIEF DESCRIPTION OF THE PREFERRED EMBODIMENTS

The present invention provides methods of biological production of plant glyceraldhyde-3-phosphate dehydrogenase by recombinant DNA technology.

The genetic sequences for plant GAPDH can be derived from any of the many source tissues therefor from any plant species. All that is required is that the genetic sequence for the protein be expressed in the host organism.

A recombinant DNA molecule comprising a genetic sequence coding for GAPDH can be used to transform a host using any of the techniques commonly known to those of ordinary skill in the art. Especially preferred is the use of a plasmid containing the GAPDH coding sequence for purposes of transformation.

Methods for preparing fused, operably-linked genes and expressing the same in bacteria are known and are shown, for example, in Riggs U.S. patent no. 4,366,246, herein incorporated by reference.

In general, expression vectors containing promoter sequences which facilitate the efficient transcription of the inserted GAPDH genetic sequences are used in connection with the host. The expression vector typically contains an origin of replication, promoter(s), terminator(s), as well as specific genes which are capable of providing phenotypic selection in transformed cells. The transformed prokaryotic hosts can be fermented and cultured according to means known in the art to achieve optimum cell growth. Examples of promoters which can be used in the invention are: rec A, trp, lac, tac, and bacteriophage lambda pR or pL. Examples of plasmids which can be used in the invention are listed in Maniatis et al., Molecular Cloning, See Cold Spring Harbor Laboratories, 1982.

For example, E. coli is typically transformed using pBR322, a plasmid derived from an E. coli species (Bolivar, et al., Gene, 2:95 (1977)). pBR322 contains genes for ampicillin and tetracycline resistance and thus provides easy means for identifying transformed cells. The pBR322 plasmid or other microbial plasmids must also contain or be modified to contain, promoters which can be used by the microbial organism for expression of its own proteins. Other promoters commonly used in recombinant DNA construction include the beta-lactamase (penicillinase) and lactose (beta-galactosidase) promoter systems (Chang, et al., Nature, 275:615 (1978); Itakura, et al., Science, 198:1056 (1977); Goeddel, et al., Nature, 281:544 (1979)) and tryptophan (trp) promoter system (Goeddel et al., Nucleic Acids Res., 8:4057 (1980); EPO Application Publication No. 0036776).

In another embodiment, the genetic sequence coding for GAPDH is linked to an inducible promoter, and the resulting genetic construct is introduced into an expression vehicle. The expression vehicle is then utilized to transform an appropriate host and the host is fermented under normal conditions wherein the promoter is not active. After an appropriate period of time, such as, for example, at a time when the cells are in stationary phase, the promoter is induced as by varying an outside environmental factor such as salt concentration, light, presence or absence of a metabolite, a metal, and the like, this change leading to transcription of the GAPDH genetic sequence into mRNA, and then translation thereof into GAPDH. Examples of inducible regulated promoters are lambda, pL and pR, lac , gal, trp, ara, hut, and the like.

For example, the genetic construct for GAPDH can be placed under the control of the leftward promoter of bacteriophage lambda (pL). This promoter is one of the strongest known promoters which can be controlled. Control is exerted by the lambda repressor, and adjacent restriction sites are known. A temperature sensitive allele of this gene can be placed on the vector that contains the GAPDH complete sequence. When the temperature is raised to 42°C, the repressor is inactivated, and the promoter will be expressed at its maximum level. The amount of mRNA produced under these conditions should be sufficient to result in a cell which contains about 10% of its newly synthesized RNA originated from the pL promoter. In this scheme, it is possible to establish a bank of clones in which a functional GAPDH fusion construct sequence is placed adjacent to a ribosome binding sequence, and at varying distances from the lambda pL promoter. These clones can then be screened and the one giving the highest yield selected.

4

Bacterial hosts are of particular interest. For example, E. coli  K12 strain 294 (ATCC 31446) is particularly useful. Other microbial strains which may be used include E. coli X1776 (ATCC 31537). The aforementioned strains, as well as E. coli W3110 (F⁻, lambda⁻, prototrophic (ATCC 27325)), and other enterobacteriaceae such as Bacillus subtilis Salmonella typhimurium or Serratia marcescens, and various Pseudomona species may be used.

The genetic constructs prepared herein and the methods involved can also be utilized for expression of GAPDH in a number of different hosts. These hosts include yeast and mammalian cell cultures. Heterologous expression in plant cells in vitro or in vivo can also be carried out. Useful yeast and mammalian hosts and vectors are well known to those of skill in the art, and reference is made, for example, to European Patent Publication 0093619 published November 9, 1983.

The invention extends to any hosts modified according to the methods described, or modified by any other methods, commonly known to those of ordinary skill in the art. such as, for example, by transfer of genetic material using a lysogenic phage, and which yield a prokaryote or eukaryote expressing the gene for plant GAPDH.

Prokaryotes transformed with the GAPDH gene sequence are of particular use for the production of GAPDH.

Any plant cell and plant capable of expressing the GAPDH gene may be transformed by this invention. These plants should also be capable of undergoing genetic manipulation by genetic engineering techniques. As used herein, the term "plant" includes plant cells, plant protoplasts, plant tissue culture that can be cultured and induced to form plants, plant calli, plant clumps and plant cells that are intact in plants or parts of plants. "Plant" also refers to pollen that may be transformed by genetic engineering techniques.

The coding region for the GAPDH that may be used in this invention may be homologous or heterologous to the plant cell or plant being transformed. It is necessary, however, that the genetic sequence coding for GAPDH be expressed, and produced, as a functional enzyme or polypeptide in the resulting plant cell. Thus, the invention comprises plants containing either homologous GAPDH genes or heterologous GAPDH genes that express the GAPDH enzyme. Further, the GAPDH may be from other plant species.

DNA from genomic DNA and cDNA encoding for the GAPDH gene may be used in this invention. Further, the GAPDH gene may be constructed partially of a cDNA clone and partially of a genomic clone. In addition, the DNA coding for the GAPDH gene may comprise portions from various plant, microbial, and animal GAPDH enzymes.

There are a variety of aspects emcompassed in this embodiment of the invention. In one of its aspects, this embodiment comprises chimeric genetic sequences with

(a) a first genetic sequence coding for a GAPDH polypeptide that upon expression of the gene in a given plant cell is functional for GAPDH activity; and

(b) one or more additional genetic sequences operably linked on either side of the GAPDH coding region. These additional genetic sequences contain sequences for plant cell promoter(s) or terminator(s). The plant regulatory sequences may be heterologous or homologous to the host cell.

Promoters that may be used in the chimeric genetic sequence include nos, ocs, and CaMV promoters.

The chimeric genetic sequence comprising the GAPDH gene operably linked to a promoter capable of operating in a plant cell can be ligated into a suitable cloning vector. In general, plasmid or viral vectors containing replication and control sequences derived from species compatible with the plant host cell are used. The cloning vector will typically carry a replication origin, as well as specific genes that are capable of providing phenotypic selection markers in transformed host cells, typically resistance to antibiotics or resistance to selected herbicides. The transforming vectors can be selected by these phenotypic markers after transformation in a host cell.

The cloning vector and host cell transformed with the vector are used in this invention typically to increase the copy number of the vector. With an increased copy number, the vectors containing the GAPDH gene can be isolated and, for example, used to introduce the chimeric genetic sequences into the plant cells.

The genetic material contained in the vector can be microinjected directly into plant cells by use of micropipettes to mechanically transfer the recombinant DNA. The genetic material may also be transferred into the plant cell by using polyethylene glycol. This forms a precipitation complex with the genetic material that is taken up by the cell. (Paszkowski et al., EMBO J., 3: 2717-22 (1984)).

In an alternate embodiment of this invention, the GAPDH gene may be introduced into the plant cells by electroporation. (Fromm et al., "Expression of Genes Transferred into Monocot and Dicot Plant Cells by Electroporation," Proc. Nat'l Acad. Sci. U.S.A., 82: 5824 (1985)). In this technique, plant protoplasts are electroporated in the presence of plasmids containing the GS genetic construct. Electrical impulses of high field strength reversibly permeabilize biomembranes allowing the introduction of the plasmids. Electroporated plant protoplasts reform the cell wall, divide, and form plant callus. Selection of the transformed plant cells with the expressed GAPDH enzyme can be accomplished using the phenotypic markers as described above.

Cauliflower mosaic virus (CaMV) may also be used as a vector for introducing the GAPDH gene into plant cells in this invention. (Hohn et al., in "Molecular Biology of Plant Tumors," Academic Press, New York, 1982 Pages 549-560; Howell, United States Patent No. 4,407,956)). The entire CaMV viral DNA genome is inserted into a parent bacterial plasmid creating a recombinant DNA molecule which can be propagated in bacteria. After cloning, the recombinant plasmid is cleaved with restriction enzymes either at random or at unique sites in the viral portion of the recombinant plasmid for insertion of the GAPDH genetic sequence. A small oligonucleotide, described as a linker, having a unique restriction site may also be inserted. The modified recombinant plasmid again may be cloned and further modified by introduction of the GAPDH genetic sequence thereof into the unique restriction site of the linker. The modified viral portion of the recombinant plasmid is then excised from the parent bacterial plasmid, and used to inoculate the plant cells or plants.

Another method of introducing the GAPDH gene into plant cells is to infect a plant cell with _Agrobacterium tumefaciens_ transformed with the GAPDH gene. Under appropriate conditions known in the art, the transformed plant cells are grown to form shoots, roots, and develop further into plants. The GAPDH genetic sequences can be introduced into appropriate plant cells, for example, by means of the Ti plasmid of _Agrobacterium tumefacianes_. The Ti plasmid is transmitted to plant cells on infection by _Agrobacterium tumefaciens_, and is stably integrated into the plant genome. Horsch et al., "Inheritance of Functional Foreign Genes in Plants," _Science_, 233: 496-498 (1984); Fraley et al, Prod. Nat'l Acad. Sci. U.S.A., 80 4803 (1983).

Ti plasmids contain two regions essential for the production of transformed cells. One of these, named transfer DNA (T DNA), induces tumor formation. The other, termed virulent region, is essential for the formation but not maintenance of tumors. The transfer DNA region, which transfers to the plant genome, can be increased in size by the insertion of the GS genetic sequence without its transferring ability being affected. By removing the tumor-causing genes so that they no longer interfere, the modified Ti plasmid can then be used as a vector for the transfer of the gene constructs of the invention into an appropriate plant cell.

All plant cells which can be transformed by _Agrobacterium_ and whole plants regenerated from the transformed cells can also be transformed according to the invention so to produce transformed whole plants which contain the transferred GAPDH gene.

There are presently two different ways to transform plant cells with _Agrobacterium_:

(1) co-cultivations of _Agrobacterium_ with cultured isolated protoplasts, or

(2) transformation of cells or tissues with _Agrobacterium_.

Method (1) requires an established culture system that allows culturing protoplasts and plant regeneration from cultured protoplasts.

Method (2) requires (a) that the plant cells or tissues can be transformed by _Agrobacterium_ and (b) that the transformed cells or tissues can be induced to regenerate into whole plants. In the binary system, to have infection, two plasmids are needed: a T-DNA containing plasmid and a _vir_ plasmid. Any one of a number of T-DNA containing plasmids can be used. The only requirement is that one be able to select independently for each of the two plasmids.

After transformation of the plant cell or plant, those plant cells or plants transformed by the Ti plasmid so that the GAPDH enzyme is expressed, can be selected by an appropriate phenotypic marker. These phenotypic markers include, but are not limited to, antibiotic resistance or herbicide resistance. Other phenotypic markers are known in the art and may be used in this invention.

All plants from which protoplasts can be isolated and cultured to give whole regenerated plants can be transformed by the present invention so that whole plants are recovered which contain the transferred GAPDH gene. Some suitable plants include, for example, species from the genera _Fragaria, Lotus, Medicago_ , _Onobrychis, Trifolium, Trigonella, Vigna, Citrus, Linum_ , _Geranium, Manihot, Daucus, Arabidopsis, Brassica, Raphanus, Sinapis, Atropa, Capsicum, Datura, Hyoscyamus, Lycopersion, Nicotiana, Solanum, Petunia, Digitalis, Majorana, Ciohorium, Helianthus, Lactuca, Bromus, Asparagus, Antirrhinum, Hemerocallis, Nemesia, Pelargonium, Panicum, Pennisetum, Ranunoulus, Senecio, Salpiglossis, Cucumis, Browallia, Glycine, Lolium, Zea, Triticum, Sorghum_, and _Datura_.

It is known that practically all plants can be regenerated from cultured cells or tissues, including but not limited to all major cereal crop species, sugarcane, sugar beet, cotton, fruit and other trees, legumes and vegetable. Limited knowledge presently exists on whether all of these plants can be transformed by Agrobacterium. Species which are a natural plant host for Agrobacterium may be transformable in vitro . Monocotyledonous plants, and in particular, cereals and grasses, are not natural hosts to Agro bacterium. Attempts to transform them using Agrobacterium have been unsuccessful until recently. Hooy-kas-Van Slogteren et al, Nature, 311:763-764 (1984). There is growing evidence now that certain monocots can be transformed by Agrobacterium. Using novel experimental approaches that have now become available, cereal and grass species may be transformed.

Additional plant genera that may be transformed by Agrobacterium include Ipomoea, Passiflora, Cyclamen, Malus, Prunus, Rubus, Populus, Santalum, Allium, Lilium, Narcissus, Ananas, Arachis, Phaseolus and Pisum.

Plant regeneration from cultural protoplasts is described in Evans, et al., "Protoplast Isolation and Culture," in Handbook of Plant Cell Culture , 1:124-176 (MacMillan Publishing Co. New York 1983) ; M.R. Davey; "Recent Developments in the Culture and Regeneration of Plant Protoplasts," Protoplasts, 1983 - Lecture Proceedings, pp. 19-29, (Birkhauser, Basel 1983) ; P.J. Dale, "Protoplast Culture and Plant Regeneration of Cereals and Other Recalcitrant Crops," in Protoplasts 1983 - Lecture Proceedings, pp. 31-41, (Birkhauser, Basel 1983) ; and H. Binding, "Regeneration of Plants," in Plant Protoplasts, pp. 21-37, (CRC Press, Boca Raton 1985).

Regeneration varies from species to species of plants, but generally a suspension of transformed protoplasts containing copies of the GAPDH gene is first provided. Embryo formation can then be induced from the protoplast suspensions, to the stage of ripening and germination as natural embryos. The culture media will generally contain various amino acids and hormones, such as auxin and cytokinins. It is also advantageous to add glutamic acid and proline to the medium, especially for such species as corn and alfalfa. Shoots and roots normally develop simultaneously. Efficient regeneration will depend on the medium, on the genotype, and on the history of the culture. If these three variables are controlled, then regeneration is fully reproducible and repeatable.

The mature plants, grown from the transformed plant cells, are selfed to produce an inbred plant. The inbred plant produces seed containing the gene for the GAPDH enzymatic activity level.

The DNA molecules of the invention or parts thereof may be labeled with a detectable group, and used as probes in hybridization assays. Such detectable group can be any material having a dectectable physical or chemical property. Such materials have been well-developed in the field of immunoassays and in general most any label useful in such methods can be applied to the present invention. Particularly useful are enzymatically active groups, such as enzymes (see Clin. Chem., 22: 1243, (1976) ), enzyme substrates (see British Pat. Spec. 1,548,741), coenzymes (see U.S. Pat. Nos. 4,230,797 and 4,238,565) and enzyme inhibitors (see U.S. Pat. No. 4,134,792) ; fluorescers (see Clin. Chem., 25:353, (1979) ; chromophores; luminescers such as chemiluminescers and bioluminescers (see Clin. Chem., 25:512, (1979)) ; specifically bindable ligands; proximal interacting pairs; and radio-isotopes such as $^3H$, $^{35}S$, $^{32}P$, $^{125}I$ and $^{14}C$. Such labels and labeling pairs are detected on the basis of their own physical properties (e.g., fluorescers, chromophores and radioisotopes) or their reactive or binding properties (e.g., enzymes, substrates, coenzymes and inhibitors). For example, a cofactor-labeled probe can be detected by adding the enzyme for which the label is a cofactor and a substrate for the enzyme. For example, one can use an enzyme which acts upon a substrate to generate a product with a measurable physical property. Examples of the latter include, but are not limited to, beta-galactosidase, alkaline phosphatase and peroxidase.

Of particular interest in this invention is the cloning and expression of chloroplast GAPDH genes. The cloned genes therefor, plasmids and expression hosts containing the same are a preferred embodiment.

Plant hosts transformed with the recombinant DNA molecules of this invention may be made resistant or les susceptible to herbicides which operate by blocking the synthesis activity of GAPDH.

The examples below are for illustrative purposes only and are not deemed to limit the scope of the invention.

EXAMPLE 1

Purification of GAPDHs

Tobacco chloroplast and cytosolic GAPDHs were isolated by a modification of the procedures developed by Cerff (1982). One hundred grams of tobacco leaves were homogenized and ammonium sulfate-fractionated as described by Cerff, (1982). To purify chloroplast isozyme I (IsoI), the 55-70% saturated ammonium sulfate fraction was further fractionated by acetone precipitation. The 0-50% acetone precipitate protein was dissolved in 19 ml TEM buffer (10 mM Tris, pH 7.8, 1mM EDTA, 14 mM 1-mercaptoethanol) and dialysed against 4.1 of the same buffer overnight. The dialysate was applied to a DEAE Sephacel column (2.5 x 10 cm) equilibrated with TEM buffer. The GAPDH activities were eluted with a 400 ml gradient of 0 to 0.3 M NaCl in TEM buffer. The active fractions were pooled and concentrated with a Millipore CX-30 immersible molecular separator. The GAPDH was further purified by two consecutive Bio-Rad A 0.5M gel filtration columns (1.5 x 100 cm) in the presence of 0.2 mM $NAD^+$ and 0.1 mM $NADP^+$, respectively (for details, see Cerff, 1982). To purify chloroplast isozyme II and cytosolic GAPDH, the 70-95% saturated ammonium sulfate fraction was desalted by passing it through a Sephadex G50 column (2.5 x 20cm) and concentrated by ultrafiltration (Amicon CFA 25). This fraction was then subjected to the gel filtration in the presence of 0.2 mM $NAD^+$. Most IsoII activity (which uses both NADPH and NADH as substrates) was eluted in the void volume, while the main peak of cytosolic GAPDH (which can only use NADH as substrate) was eluted in the fractions of $V_e/V_o = 1.6$. The fractions containing IsoII were concentrated and further purified by $NADP^+$ gel filtration. The fractions containing the cytosolic enzyme were pooled and dialyzed against HA buffer (20 mM $KHPO_4$, pH 7.4, 1 mM EDTA, 5 mM 2-mercaptoethanol) and applied to a Hydroxylapatite column (1.5 x 25 cm) equilibrated with HA buffer. The GAPDH was then elu ted by a salt gradient containing from 20 mM to 0.2 M $KHPO_4$.

Anti-$A_2B_2$ and Anti $C_4$ Antibodies

Each of two New Zealand White Rabbits were injected with 200ug purified $A_2B_2$ or $C_4$ GAPDH in complete Freund's adjuvant. Booster injections were administered four weeks after the initial injection. The rabbits were bled one week after the booster injection. The antisera were then purified by ammonium sulfate precipitation (0 - 50% saturation) and dialyzed against a buffer containing 0.9% NaCl, 10 mM sodium phosphate, ph 7.2 and stored frozen.

N-terminal Amino Acid Sequence Analyses

Protein (200 ug) from each purified isozyme was precipitated by adding 4 volumes of methanol/acetone (V/V = 1:1). The pellet was dried and resuspended in 100 ul 1.5% triethylamine (HPLC grade). The sample was then subjected to sequence analysis in an automatic Applied Biosystem 470A Protein Sequencer.

EXAMPLE 2

Hybrid selection was performed according to the procedure described by Miller et al., Methods in Enzymology, 101:650 (1983), using the protocol for the highly abundant mRNA with modification of increasing the stringency of washing to 0.1 x SSC, 0.5% SDS at 70°C.

EXAMPLE 3

In vitro Translation and Immunoprecipitation

Rabbit reticulocyte lysate (Bethesda Research Laboratories) was used for in vitro translation according to the supplier's manual. For immunoprecipitation, the antiserum was conjugated to protein-A sepharose CL-48 (Pharmacia Inc.) in the presence of 50 mM $KHPO_4$, pH 7.4, 0.5 m NaCl for 2 hours at 4°C. The gel was washed three times with a buffer containing 25 mM Tris, pH 7.6, 0.15 M NaCl, 1 nM EDTA, and 0.1% SDS. The in vitro translated product was then incubated with antiserum-protein-A sepharose complex at 4°C for 2 hours and washed 4 times with the same buffer. The immunoprecipitated proteins were eluted by boiling and directly loaded onto 10% SDS polyacrylamide gels.

## EXAMPLE 4

### Screening of cDNA Libraries

The cDNAs were synthesized according to the procedure of Gubler and Hoffman, Gene, 25:626 (1983). The cDNAs were C-tailed and annealed to G-tailed pUCl2 vectors and transformed into E. coli strain MCl061. The procedure of Hannahan and Meselson, Gene, 10:63 (1980) was used to screen cDNA libraries. The oligonucleotides were end-labelled with [gamma-P32] ATP (>3000 Ci/mmol) by T4 polynucleotide kinase and used as probe. The hybridization was done at 5°C below $T_m$(Wallace et al., 1981) in 6 x NET (1 x NET = 0.15 M NaCl/1 mM EDTA/15 mM Tris-HCl, pH 7.5), 5 x Denhardt's solution, 0.5% SDS, 100 ug/ml tRNA, 10% Dextran sulfate for 16 to 24 hours. The filters were washed twice at room temperature 15 min each in 6 x SSC and four times at 3 to 5°C below $T_m$.

### DNA Sequence Determination

The cDNA fragments were subcloned into M13 and mp18 or 19 and sequenced by the dideoxynucleotide chain termination method (Sanger et al., Proc. Natl. Acad. Sci. USA, 74:5463 1977).

Plasmid DNA was prepared by the alkaline method (Birnboim and Doly, Nuc. Acid Res., 7:1513 1979) and purified by CsCl gradient centrifugation twice. Tobacco RNA was isolated by a modification of the phenol extraction method (Maniatis et al., 1982).

## EXAMPLE 5

### Purification of GAPDH from Tobacco

The cytosolic ($C_4$) and chloroplast ($A_4$ and $A_2B_2$) holoenzymes for GAPDH were purified from Nicotiana tabacum W38 to greater than 90% purity (as described in Example 1). The molecular weights as determined by SDS-polyacrylamide gel electophoresis for the A, B and C subunits are 37,000 d., 41,000 d., and 36,000 d., respectively. All three holoenzymes were subjected to N-terminal sequence analysis. The N-terminus of the $C_4$ enzyme is blocked, however, N-terminal sequences of 17 and 30 amino acids, respectively, were obtained from $A_4$ and $A_2B_2$ (Table 1a). Surprisingly, the $A_2B_2$ enzyme gave a unique sequence, the first 17 residues of which are identical to those of the $A_4$ protein. This later result indicates that the N-terminus of the B subunit is blocked or that the A and B subunits have the same N-terminal sequences. The recovery of lysine in the first cycle of the sequencing reaction was 65% suggesting the second possibility since this rarely exceeds 50% for single polypeptide chains (cf. nucleotide sequence data below). A block of seven amino acids, Gly-Phe-Gly-Arg-Ile-Gly-Arg, which is conserved among all cytosolic GAPDHs (Tso et al., 1985a) is also present near the N-terminus of the chloroplast enzymes (Table 1).

## EXAMPLE 6

### Screening of Tobacco cDNA Libraries for GAPDH

Based on the N-terminal amino acid sequences from tobacco (Table 1) and the conserved sequences among the cytosolic GAPDHs from other species (Tso et al., 1985a) four oligonucleotides were synthesized (Table 2). The 15mer (15.1) and 23mer (23.1) were deduced from amino acid sequences conserved among all the GAPDHs whose peptide sequences are known, while the two 18mers (18.1 and 18.2) were deduced from a region in the N-terminal sequence of the tobacco chloroplast enzymes which does not show any homology with known cytosolic GAPDH sequences (Table 2). Therefore, it was anticipated that potential full length cDNA clones for the genes encoding the A and B subunits (GapA and GapB) should hybridize to three oligonucleotides (15.1; 18.1 or 18.2; and 23.1), while cDNAs for the cytosolic GAPDH gene (GapC) should hybridize to 15.1 and 23.1, but not to 18.1 or 18.2. Indeed, when these oligonucleotides were used as probes to screed cDNA libraries made from leaf RNA of tobacco plants grown under normal green house conditions and suspension cultures of leaf callus grown in complete darkness (under this condition the chloroplast is in the undifferentiated state), three classes of cDNA clones were obtained (Fig. 3). Clones with insert sizes larger than 1.2 kb from all three classes hybridized to both 15.1 and 23.1. Class I clones hybridized to 18.1 and class II to 18.2, while class III clones do not hybridize to either 18.1 or 18.2. Classes I and II were only present in the cDNA library obtained from light grown tobacco, while class III could be obtained from both light and dark grown tobacco cDNA libraries. These results suggest that class I and II contain the cDNAs encoding the GapA and GapB genes, while class III contains those corresponding to the GapC gene. Plasmids containing the GapA, GapB, and GapC genes have been deposited in accordance with the Budapest treaty with the American Type Culture Collection and assigned deposit numbers 67225, 67226, and 67224, respectively.

## EXAMPLE 7

### Characterization of cDNA Clones

Hybrid selection was used to further characterize these cDNA clones; poly A$^+$ RNA isolated from tobacco leaves was hybrid selected and after in vitro translation the products were separated by SDS-polyacrylamide gel electrophoresis. When pND32 (a class III clone) was used to hybrid select RNA, the in vitro translation product was a polypeptide with the same molecular weight (36,000 d.) as that of purified cytosolic GAPDH. This protein was immunoprecipitated by antiserum raised against cytosolic GAPDH, hence, class III consists of the cDNA clones encoding the cytosolic peptide, GapC. In contrast, when pG49 (a class I clone) and pG60 (a class II clone) were used to hybrid-select RNA, the in vitro translation products were peptides with molecular weights of 43,000 and 47,000 d., respectively. Both of these peptides can be immunoprecipitated by antiserum raised against the holoenzyme A$_2$B$_2$, confirming the assignment of class I and II as GapA and GapB clones.

The molecular weights of the in vitro translation products are larger than those of the A and B subunits of the purified chloroplast GAPDH by 6,000 d. This suggests, by analogy to other nuclear coded chloroplast proteins, that the A and B subunits contain N-terminal transit peptides of 60 amino acids each. In addition, the differences in the molecular weights of the in vitro translation products and the holoenzyme subunits indicates that class I encodes the GapA and class II and GapB peptides, respectively.

There is an in vitro translation product in the absence of added poly A$^+$ RNA that comigrates with the A peptide precursor. However, immunoprecipitation results indicate that the majority of the 43,000 d. protein band is the translation product of the RNA hybrid-selected by clone pG49.

The complete nucleotide sequences of three clones (pG49, pG60, and pNDA1) which were the longest obtained from classes I, II, and III, respectively were determined (Table 4a). All three clones contain features characteristic of GAPDH which are conserved throughout evolution (Tso et al., 1985a). The N-terminal domains of the deduced peptide sequences of both pG49 and pG60 contain a 30 amino acid sequence (positions 1-30, Table 4b) that is exactly the same as the sequence determined from purified chloroplast GAPDH (Table 1). There are open reading frames of 58 and 53 amino acids preceding the N-terminal codon of the mature peptide from pG49 and pG60, respectively. These data are consistent with the conclusion that pG49 and pG60 correspond to the chloroplast clones; the open reading frames preceding the first codon of the mature protein encode the transit peptide.

The calculated molecular weights for the mature peptides coded by pG49 and pG60 are 36,000 and 41,000 d., respectively. These values correspond well with those of the A (MW = 37,000 d.) and B (MW = 41,000 d.) subunits determined from SDS-polyacrylamide gel electrophoresis. The deduced amino acid sequence of the GapC clone, NDA1, indicates that it is missing the first 9 amino acids of the C subunit (Table 4).

## TABLE 1

A$_4$    Lys·Leu·Lys·Val·Ala·Ile·Asn·Gly·Phe·Gly·Arg·Ile·Gly·Arg·Asn·Phe·Leu

A$_2$B$_2$    Lys·Leu·Lys·Val·Ala·Ile·Asn·Gly·Phe·Gly·Arg·Ile·Gly·Arg·Asn·Phe·Leu

Arg·Ser·Trp·His·Gly·Arg·Lys·Asp·Ser·Pro·Leu·Asp·Val·Ile·Ala

## TABLE 2

NH$_2$ ——— INGFGRIGR ——— WHGRKD ——————— WYDNE ——— COOH
　　　　　　　23.1　　　　　　18.1　　　　　　　　　　15.1
　　　　　　　　　　　　　　　18.2

23.1  CTACCAATCCTACCAAAACCATT
　　　　　　T

18.1  ATCCTTCCTNCCATGCCA
　　　　　T  T　　　G

18.2  ATCATTNCGNCCCTGCCA
　　　　　G　　　　　　T

15.1  CTCATTATCATACCA
　　　　　G　　G　G

## TABLE 3

|         | 23.1 * | 18.1 * | 18.2 * | 15.1 * |
|---------|--------|--------|--------|--------|
| Class I   | +    | +      | −      | +      |
| Class II  | +    | −      | +      | +      |
| Class III | +    | −      | −      | +      |

# TABLE 4a-I

```
preA  AACTCTTCT CTTCAGGTCA GCAACAAAGG ATTCTCTGAA TTCTCAGGGC TGCGCACCTC ATCAGCTATT CCATTCGGAA
preB            TGCTTATCCA AGAAATTTGA AGTAGCCGAA TTTGCTGGTC TTCGATCGAG TGGATGTGTG ACATTTTCCA

      GGAAAACTAA CGATGACTTG CTCTCTGTTG TTGCCTTTCA AACCTCTGTT ATTGGAGGAG GGAACAGCAA GAGGGGAGTA
      ACAAAGAGTC TTCTTTCTTT GATGTTGTCT CTGCTCAACT CACTCCAAAG ACCACAAGAT CAACCCCTGT TAAGGGAGAA

      GTGGAGGCC
      ACTGTTGCT

GapA  AAGTTGAAAG TGGCAATCAA TGGATTTGGA AGAATTGGGA GGAATTTCTT GAGGTGTTGG CATGGTAGGA AAGACTCTCC
GapB  AAATTGAAGG TTGCTATCAA TGGTTTTGGA CGAATTGGTA GGAATTTCCT CCGATGCTGG CATGGGCGCA AAGACTCGCC
GapC                           GGT AGAATTGGCC GTTTGGTGGC AAGAGTTGCT CTGCAGAGAG ATGATGTTGA

      CCTTGATGTC ATTGCCATCA ATGAC...AC TGGTGGTGTC AAGCAAGCCT CTCACCTTCT TAAATATGAC TCCACCCTTG
      ACTAGATGTC GTGGTTGTCA ACGAC...AG TGGTGGTGTC AAGAATGCAT CTCACTTGCT AAAGTATGAT TCGATGCTGG
      ACTA...... GTTGCAGTGA ACGACCCATT TATCTCTACT GATTACATGA CATACATGTT TAAGTATGAT TCGGTTCATG

      GCATCTTTGA TGCTGATGTC AAGCCCGTCG GCACTGACGG CATCTCCGTC GACGGA.... ..AAAGTCAT CCAAGTCGTC
      GAACATTCAA AGCTGATGTG AAAATAGTGG ATAATGAAAC CATTAGTGTT GATGGA.... ..AAGCACAT CAAGGTTGTT
      GACAATGGAA ACACCATGAG CTTAAAGTAA AGGATGAA.. .AAGACCCTT CTTTTTGGTG AGAAGTCCGT CAGAGTCTTT

      TCCGATCGCA ACCCCGTCAA CCTCCCCTGG GGAGATCTTG GGATTGACTT GGTGATAGAA GGTACCGGAG TGTTTGTCGA
      TCTAGCAGGG ACCCCCTTAA GCTTCCTTGG GCTGAACTTG GCATCGACAT TGTTATTGAG GGAACCGGTG TGTTTGTTGA
      GGAATTAGGA ACCCTGAGGA AATTCCATGG GCTGAAGCTG GTGCTGATTT CGTTGTGGAA TCCACTGGTG TCTTCACTGA
```

0 264 067

# TABLE 4a-2

```
CAGAGAAGGC GCCGGAAAAC ACATTCAGGC TGGAGCCAAG AAGGTGCTCA TCACCGCTCC CGGAAAAGGT ...GACATCC
TGGTCCAGGT GCTGGGAAGC ACATCCAAGC TGGTGCCAAG AAAGTTATCA TCACTGCACC AGCAAAAGGT GCTGACATTC
CAAGGACAAG GCTGCTGCTC ACTTGAAGGG TGGTGCCAAG AAGGTTGTGA TCTCTGCTCC TAGCAAG... ...GATGCCC

CCACATATGT TGTTGGTGTC AATGCT...G ATCTCTACAA CCCTGATGAA CCTATCATCA GCAATGCCTC TTGCACCACC
CTACCTATGT CGTTGGGGTG AACGAGCAAG ACTACTCTCA CGAGGTTGCT GATATCATAA GCAATGCCTC TTGCACCACT
CCATGTTTGT TGTGGGTGTC AACGAG...A AGGAATACAA GCCCGAATAT GACATTGTCT CCAATGCTAG TTGCACTACC

AACTGCCTTG CTCCTTTTGT CAAGGTTCTT GACCAAAAT TCGGCATTAT CAAGGGAACC ATGACAACTA CTCACTCTTA
AACTGCTTGG CTCCATTTGT AAAGGTCATG GATGAAGAAC TTGGTATTGT GAAGGGTACA ATGACAACAA CTCACTCTTA
AACTGCCTTG CACCTTTGGC TAAGGTCATC AATGATAGGT TTGGCATTGT GGAGGTCTC ATGACTACTG TGCACTCCCT

CACCGGTGAC CAAAGGCTTC TTGAT...GC GAGCCACAGG GACCTTAGAC GTGCACGAGC TGCAGCCCTC AACATCGTTC
CACCGGAGAT CAGAGGCTTC TAGAT...GC TTCACACCGT GACTTGAGGA GAGCGAGAGC AGCAGCATTG AACATTGTCC
TACTGCCACC CAGAAGACTG TTGATGGTCC ATCCATGAAG GACTGGAGAG GTGGAAGAGC TACTTCATTC AACATCATTC

CAACCTCAAC TGGTGCTGCT AAGGCCGTGG CCTTGTCCTC CCAAGCCTTA AGGGGAAGCT CAATGGCATT GCCCCTCCGT
CAACCAGCAC TGGTGCAGCC AAGGCTGTGT CTTTAGTGCT ACCTCAACTT AAGGGAAAGC TCAATGGCAT TGCTCTCCGT
CTAGCAGCAC TGGTGCTGCC AAGGCTGTTG GAAAAGTGCT CCCAGCTCTT AATGGAAAAT TGACTGGAAT GGCCTTCAGA

GTGCCAACCC CTAACGTCTC GGTCGTTGAC CTTGTCGTCC AAGTCTCCAA GAAG...ACA TTTGCTGAGG AAGTGAATGC
GTCCCAACAC CTAATGTTTC AGTTGTTGAC CTCGTTGTCA ATGTCGCGAA GAAAGGAATT ACAGCTGAGG ATGTCAATGC
GTTCCAACTG TTGATGTTTC TGTTGTGGAC CTTACCGTAA GACTAGAGAA AGAA...GCC TCTTATGATG ACATCAAAGC
```

# TABLE 4a-3

```
TGCATTTAGG GAGGCTGCTG ACAAGGAGCT CAAAGGCATT CTCGATGTCT GCGATGAACC ACTCGTGTCA GTCGACTTCC
AGCTTTCAGA AAAGCTGCTG ATGGTCCATT GAAAGGCGTA TTAGCTGTGT GCGATGAACC TCTTGTTTCA GTTGACTTCA
TGCAATCAAG GAGGAATCGG AGGGTAAGTT GAAGGGCATC TTGGGATTCA CCGAAGATGA TGTGGTTTCC ACAGACTTTG

GGTGCAGTGA TGTGTCATCA ACTGTTGATG CTTCACTTAC TATGGTCATG GGAGATGACA TGGTTAAGGT TATTGCTTGG
GATGCTCTGA TGTCTCATCC ACCATCGACT CCTCTTTAAC CATGGTTATG GGAGACGATA TGGTCAAGGT CGTGGCTTGC
TTGGTGACAG CAGGTCAAGC ATTTTCGATG CCAAGGCTGG AATTGCCTTG AGCAAGAACT TTGTGAAACT TGTGTCGTGG

TATGACAATG AATGGGGTTA CTCACAGAGG GTGGTTGATC TTGCTGACAT TGTTGCAAAC CAGTGGAAAT GAaatatgaa
TACGATAACG AGTGGGGATA CAGCCAACGT GTGGTGGATT TGGCTCATCT AGTAGCAAAC GTAGCAAACA ATTGGCCAGC
TATGACAATG AATGGGGTTA CAGCTCTCGT GTGATTGATT TGATCTGCCA TATGGCTTCT GTTGCTTAAg gatttgatgc

ataacagtac tgctaaatag ttgcttaaat tttttccact gcttgtagat tattattcct ttttgaaaat tgtaatgaga
AAGTTGCAGT ACCAGGAAGT GGAGATCCCA TTGGATGAGT TTTGCAAGAC AAATCCTGCT GATGAGGAAT GCAAAGTCTA
tggtgaaatg gcctctttag tttttgattg aatcataggg gtattagttt tctatggccg ggagtggtct tcttgcttaa

accaaaacaa ttttccacct tgtgaagtct cagcttcaat aaaagtttcc ttcttgtctt aaaaaaaaa
TGAATAATCG TTTTTGTTAT GTTTCCTCTT TTGAAATGGA TGATTTTAGA AGCCAATAAt tttttgtct acagatttag
ttgtaatgga ataaccagag aggaactact gtgttatctt tgaggaatgt tgggcttttt tcgtttgaat tatcatgaat
```

GapB tctgtaaaac ttagtggcaa attaccacta at
GapC gaaattttac tttttcccaa ttaaaaaaaa aaaaaaaaa aaaa

0 264 067

# TABLE 4b

```
trnA                                                                      NSSLQV
trnB                                                                         CLS

trnA  -50   SNKGFSEFSG LRTSSAIPFG RKTNDDLLSV VAFQTSVIGG GNSKRGVVEA
trnB  -50   KKFEVA  A     S GCVT S  N ESSFFDV  S QL PKTTR STPVK ETV

GapA   1    KLKVAINGFG RIGRNFLRCW HGRKDSPLDV IAINDxTGGV KQASHLLKYD
GapB   1                                     VVV   xS      N
GapC   1    ----------    LVA VA LQ D VE xx VAV   PFIST DYMTYMF

GapA   50   STLGIFDADV KPVGIDGISV DGxxKVIQVV SDRNPVNLPW GDLGIDLVIE
GapB   50   M  T K      I DNET      xx H K    S D LK     AE     I
GapC.  49   VH QWKHHE LK KDExKTL LFGE SVR F GI N EEI   AEA A F V

GapA   98   GTGVFVDREG AGKHIQAGAK KVLITAPGKG xDIPTYVVGV NAxDLYNPDE
GapB   98          GP                 I    A  A          EQ YSHEVA
GapC   99   S    T KDK  AA LKG     V S  S x  x A MF      ExKE KPEY

GapA   148  PIISNASCTT NCLAPFVKVL DQKFGIIKGT MTTTHSYTGD QRLLDxASHR
GapB   148  D                         M EEL   V                     x
GapC   148  D V               LA  I NDR   VE L·    V  L AT  KTV GP MK

GapA   195  DLRRARAAAL NIVPTSTGAA KAVALSSQAL RGSSMALPLR VPTPNVSVVD
GapB   197                               S VLPQ  K KLNGIA
GapC   196     W GG   TSF   I S      GKVLP  N KLTGMAF      VD

GapA   249  LVVQVSKKxT FAEEVNAAFR EAADKELKGI LDVCDEPLVS VDFRCSDVSS
GapB   247       N A  GI T D       K   GP   V  A
GapC   246   .  T RLE ExA SYDDIK  IK  ESEGK      GFTEDDV    T  VGDSR

GapA   294  TVDASLTMVM GDDMVKVIAW YDNEWGYSQR VVDLADIVAN QWK*
GapB   297  I S                V               HL     NWPGSCSTRK
GapC   295  IF  KAGIAL SKNF  LVS        S   I  ICHM S VA*


GapB   347  WRSHWMSFAR QILLMRNAKS MNNRFCYVSS FEMDDFRSQ*
```

## Claims

1. A recombinant DNA (rDNA) molecule which contains a plant-derived genetic sequence for glyceraldehyde phosphate dehydrogenase.

2. The rDNA molecule of claim 1 wherein said genetic sequence codes for chloroplast glyceraldehyde phosphate dehydrogenase.

3. The rDNA molecule of claim 1 wherein said genetic sequence codes for cytosolic GAPDH.

4. The rDNA molecule of any of claims 1-3, wherein said genetic sequence is in cDNA form.

5. The rDNA molecule of any of claims 1-3 wherein said plant is tobacco.

6. The rDNA molecule of any of claims 1-3 wherein said molecule is a vector.

7. The rDNA of claim 6 wherein said vector is a plasmid.

8. A host transformed with the rDNA of any of claims 1-3.

9. A host according to claim 8 which is a bacterium.

10. A host according to claim 8 which is a yeast.

11. A host according to claim 8 which is a mammalian cell.

12. A host acording to claim 8 which is a plant cell.

13. A method of producing plant glyceraldehyde phosphate dehydrogenase which comprises:

(a) providing the DNA molecule of any of claims 1, 2 or 3;

(b) inserting said molecule into a vector;

(c) transforming a host system with said vector;

(d) expressing said GAPDH DNA sequence of said DNA molecule in said host; and

(e) isolating the plant glyceraldehyde phosphate dehydrogenase produced by said expression.

14. A method of producing plant glyceraldehyde phosphate denydrogenase according to claim 13 wherein the glyceraldehyde phosphate dehydrogenase is from chloroplasts.

15. A method of producing plant glyceraldehyde phosphate denydrogenase according to claim 13 wherein the glyceraldehyde phosphate dehydrogenase is from cytosol.

16. The recombinant DNA molecule of claim 1 which contains a detectable lable.

17. The recombinant molecule of claim 16 wherein the detectable label is selected from a radiolabel, a fluorescing label, a chemiluminescing label, an enzyme label and a free radical label.

Claims for the following contracting state: ES

1. A method of producing plant glyceraldehyde phosphate dehydrogenase (GAPDH) which comprises:

(a) providing a recombinant DNA (rDNA) molecule which contains a plant-derived genetic sequence for GAPDH;

(b) inserting said molecule into a vector;

(c) transforming a host system with said vector;

(d) expressing said GAPDH DNA sequence of said DNA molecule in said host; and

(e) isolating the plant GAPDH produced by said expression.

2. A method of producing plant GAPDH according to claim 1, wherein the GAPDH is from chloroplasts.

3. A method or producing plant GAPDH according to claim 1, wherein the GAPDH is from cytosol.

4. The method of any of claims 1 - 3, wherein said genetic sequence is in cDNA form.

5. The method of any of claims 1 - 3, wherein said plant is tobacco.

6. The method of any of claims 1 - 3, wherein said rDNA molecule is a vector.

7. The method of claim 6, wherein said vector is a plasmid.

8. The method of claim 1, wherein the rDNA molecule contains a detectable label.

9. The method of claim 8, wherein the detectable label is selected from a radiolabel, a fluorescing label, a chemiluminescing label, an enzyme label and a free radical label.

10. A method for transforming a host which comprises introducing into the host cell a rDNA of any of claims 1 - 9.

11. A method according to claim 10, wherein the host is a bacterium.

12. A method according to claim 10, wherein the host is a yeast.

13. A method according to claim 10, wherein the host is a mammalian cell.

14. A method according to claim 10, wherein the host is a plant cell.